(19) Europäisches Patentamt
European Patent Office
Office européen des brevets

(11) Publication number: 0 116 104 A1

# (12) EUROPEAN PATENT APPLICATION

(21) Application number: 83101338.8

(22) Date of filing: 11.02.83

(51) Int. Cl.³: G 01 N 33/24
G 01 N 31/00

(43) Date of publication of application:
22.08.84 Bulletin 84/34

(84) Designated Contracting States:
AT BE CH DE FR IT LI LU NL SE

(71) Applicant: Arthur Yates and Co. Limited
270 Neilson Road
Onehunga Auckland(NZ)

(72) Inventor: Prasad, Munoo
Kimberley Road
Levin(NZ)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

## (54) Method and arrangement for nutrient testing.

(57) The invention relates to a method for testing mineral soils and soiless compost to determine the nutrient content thereof. The subject matter provides for the extraction of one or more nutrients from growing medium including mixing together the growing medium and extractant, thereafter shaking or agitating the mixture by hand for a predetermined period of time. The method provides for a filtrate of such a mixture to be tested for the presence of one or more nutrients.

EP 0 116 104 A1

This invention relates to methods and arrangements for testing mineral soils and soiless compost to determine the nutrient content thereof.

Numerous mineral soils and soiless composts do not have the ability to retain minerals and nutrients, and it is thus important to have the ability to regularly test and check such mineral soils and soiless compounds for nutrients and nutrient levels. This then enables mineral and nutrient feeding to maintain adequate and desired levels. It is also of course important to be able to monitor nutrient status of various mediums in order to ensure that plants can grow under optimum conditions, whether they be cropping plants such as for example tomatoes, or shrubs.

Up until this time, various means for the analysis of substrates have been put forward and provided, but these have all entailed to a large extent, analysis in laboratories or laboratory conditions, which has involved delays in having tests carried out and has also involved reasonably substantial expense. In particular, in the case of horticulturalists involved for example in growing plants or shrubs, it becomes very time consuming and expensive to have to deliver samples of substrate to laboratories and then go and pick them up at a later date following analysis. Such analyses can often take two or three days, and can be expensive, and in addition, do not provide instant or immediate analysis. As will be appreciated if analysis in a laboratory situation takes something like two or three days, by the time the analysis is returned, the state of the substrate initially analysed will have changed. The present invention sets out to provide a method and arrangement whereby mineral soils and soiless composts (hereinafter referred to as "substrate") can be analysed substantially insitu, or on site. The invention provides a kit for the analysis of substrate to determine nutrient presence and also provides for a method of carrying out such testing.

By using the present invention users are able to carry out testing themselves and are able to obtain results at the time of carrying out the tests, thus overcoming the problems encountered with analysis and testing up until this time. It will also be appreciated that by permitting users to carry out testing themselves, more regular and frequest testing and analysis can take place, thus keeping a more regular or frequent monitor on the nutrient state of the substrate.

The present invention has application to the analysis or testing of substrates such as soiless growing media such as compost, peat, bark, pumice and sawdust mixes. It also has application to nutrient solutions of a nutrient film technique and has application also to mineral soils.

The present invention provides relative accuracy in testing, and provides apparatus within the arrangement and method of the invention to permit straight forward and efficient testing and analysis to take place.

SUMMARY OF THE INVENTION

According to one aspect of this invention, there is provided a method of extracting one or more nutrients from a substrate, said method including mixing together said substrate with an extractant and thereafter shaking or agitating said mixture by hand for a predetermined period of time.

According to a further aspect of this invention, there is provided a method of extracting one or more nutrients from a substrate including mixing together said substrate with an extractant thereafter shaking or agitating said mixture by hand for a period of up to two minutes.

According to a further aspect of this invention, there is provided a method of testing a filtrate of a mixture of substrate and extractant for nitrate nutrients,said filtrate having been extracted by the preceding method including:

1. inserting at least one strip of nitrate test paper into said filtrate and thereafter withdrawing same.
2. inserting at least one strip of nitrate test paper into at least one nitrate standard (as herein defined) and thereafter withdrawing same.
3. waiting for a predetermined period of time.
4. thereafter comparing colours of said test strips.

According to a further aspect of this invention, there is provided a method of testing a filtrate of a mixture of substrate and extractant for presence of phosphorus nutrients said filtrate having been extracted according to the method according to the preceding paragraphs, including:

1. taking one or more standard containers.
2. taking one or more sample containers.

3. inserting ½ ml of said filtrate into said one or more sample containers.

4. inserting ½ ml of phosphorus standard (as herein defined) in said one or more standard containers.

5. inserting 2 mls of phosphorus re-agent into each container.

6. waiting for a predetermined period of time.

7. thereafter comparing the colours of said one or more sample containers with the colour of said one or more standard containers.

According to a further aspect of the invention there is provided a method of testing for the presence of potassium nutrients in a filtrate, having been extracted from a mixture of substrate and extractant according to the method outlined in the preceding paragraphs, including:

1. inserting ½ ml of filtrate into one or more sample containers.

2. inserting ½ ml potassium standard (as herein defined) into one or more standard containers.

3. inserting a predetermined amount of first potassium re-agent into each container.

4. inseting predetermined amount of second potassium re-agent into each container.

5. inserting predetermined amount of third potassium re-agent into each container and thereafter mixing each container.

6. allowing said containers to sit for a predetermined period of time.

7. after expiration of said predetermined period of time comparing cloudiness of said one or more sample containers with cloudiness of said one or more standard containers to determine standard of potassium nutrients present in said filtrate.

According to a further aspect of this invention, there is provided a method of testing for the presence of ammonium nutrient in a filtrate of a mixture of substrate and extractant said filtrate being extracted from said mixture of substrate and solvent by a method outlined in any one of the preceding paragraphs, including:

1. inserting ½ ml of said filtrate into a sample container.

2. inserting ½ ml of nitrogen standard (as herein defined) into one or more standard containers.

3. inserting a predetermined amount of water into each container.

4. inserting 2 ml of ammonium re-agent into each container and thereafter closing and shaking same .

5. after a predetermined period of time, comparing colour in said one or more sample containers with the colour in one or more standard containers to determine ammonium nutrient presence.

According to a further aspect of this invention, there is provided an arrangement for testing substrate for one or more nutrients, including:

1. at least one open ended container.

2. a mixture of substrate and extractant being provided within said container.

3. at least one filter being provided and located within said container so as to extend into said mixture.

4. a filtrate being defined by said filter within said container.

5. said filtrate being an extractant from said mixture of substrate and extractant.

6. said filtrate being separated from said mixture of substrate and extractant but within said container, by location of said filter.

According to a further aspect of this invention, there is provided apparatus for use in testing substrate for one or mo: nutrients, including a casing or housing provided with or housing and locating:

1. one or more shaker containers;

2. at least one primary container;

3. at least one filter capable of being located within said primary container, such as to define a filtrate within said container

4. a plurality of secondary containers or tubes such as to provide one or more sample containers or one or more standard containers for nutrient tests;

5. a plurality of nutrient standard containers (as herein defined).

6. a plurality of nitrate test strips.

7. phosphorus re-agent stock (as herein defined).

8. abscorbic acid.

9. first, second and third potassium re-agents (as herein defined).

10. one or more applicators.

BRIEF DESCRIPTION OF THE DRAWINGS

This invention will now be described by way of example only, and with reference to the accompanying drawings wherein:

Figures 1, 1A & 1B: are diagrammatic and schematic views of the mixing of the substrate and extractant and extraction of the filtrate for use in testing.

Figure 2: is a diagrammatic and schematic view of testing for nitrate nutrients.

Figure 3: is a diagrammatic and schematic view of testing for phosphorus nutrients.

Figure 4: is a diagrammatic view for testing for potassium nutrients.

Figure 5: is a diagrammatic and schematic view of testing for ammonium nutrients.

DESCRIPTION OF THE PREFERRED EMBODIMENTS

Throughout the specification and claims, certain terms will be used and for assistance in reading and interpret-

ation, and without prejudice to the generality of the normal interpretation of such terms, the terms will be hereinafter defined as follows:

The term "substrate"is hereinafter defined as including mineral soils and compost including soiless compost, such as for example peat, bark, pumice, sawdust mixes.

The term "standard container", is hereinafter defined as a container for the use in determining standards of nutrient levels for use in comparison and testing.

The term "sample container" is hereinafter defined as a container used for holding or having mixed therein a sample of filtrate and one or more additives for use in testing for the presence of one or more nutrients in said filtrate.

The term "filtrate" is hereinafter defined as that matter extracted from a mixture of substrate and extractant and which has been filtered by a filter so as to be separated from a main mixture of substrate and solvent.

The term "shaker container" is hereinafter defined as a container used for shaking and agitating matter and is for example used to mix together by shaking and agitation, substrate and extractant.

The term "primary container" is hereinafter defined as a container in which a mixture of substrate and extractant is placed after shaking or agitating in which a filter is preferably located for the separation of a filtrate (as hereinbefore defined) from the mixture of substrate and extractant.

The term "nitrate test paper" is hereinafter defined as testing paper preferably in strips, and divided into sections for use in testing nitrogen or nitrate concentrations, which are indicated by the reaction of the strip paper, such as by colours being activated.

The term "nitrogen standard" is hereinafter defined as predetermined amounts of nitrogen or matter containing predetermined amounts of nitrogen for use in comparison with amounts of nitrogen in samples tested in sample containers (as herein defined).

The term "phosphorus re-agent stock solution" is hereinafter defined as a mixture of ammonium molybdate, antimony potassium tartartate and sulphuric acid.

The term "phosphorus re-agent tablet" where used is hereinafter defined as an ascorbic acid tablet or tablet including ascorbic acid.

The term "phosphorus re-agent" is hereinafter defined as a combination of re-agent stock solution, (as herein defined) and ascorbic acid or phosphorus re-agent tablet (as herein defined).

The term "phosphorus standard" is hereinafter definec as matter being or including predetermined amounts of phos phorus to establish standards of phosphorus content agains which tests or comparisons can be made.

The term "potassium standard" is hereinafter defined matter being or including predermined amounts of potassiu for use in testing or comparison to determine potassium levels in samples.

The invention has particular application to the analysis or testing of substrate to determine nutrient levels and in particular nitrate, ammoniacal nitrogen, phosphorus and potassium levels.

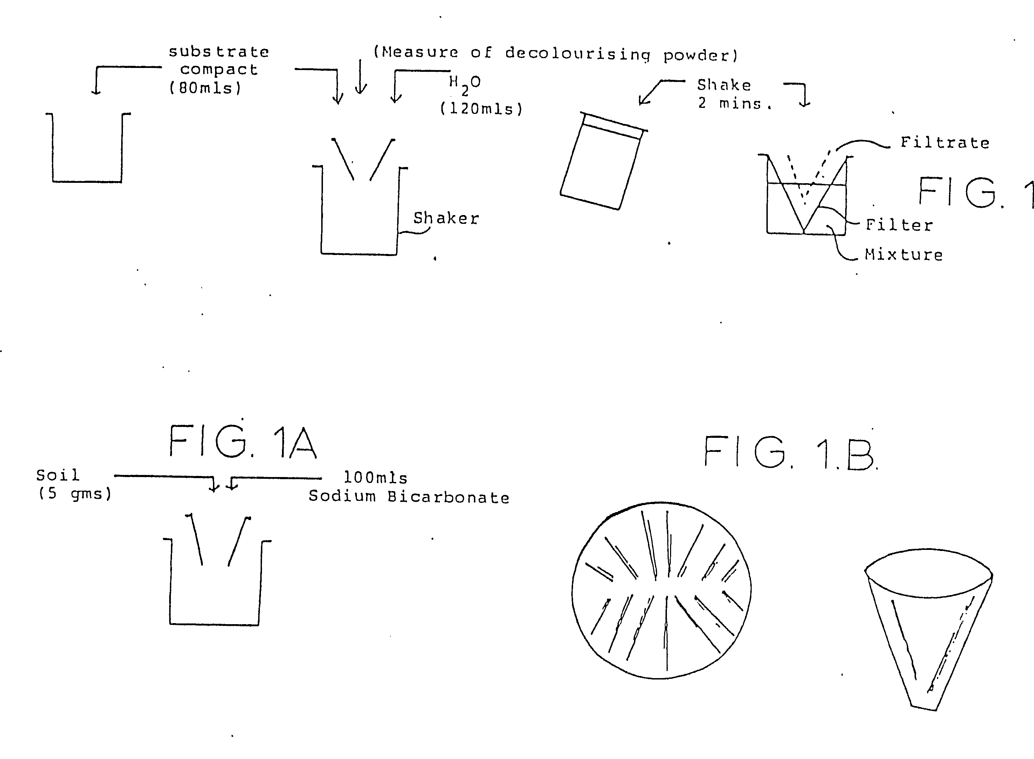

Referring firstly to Figure 1 of the accompanying drawings, a sample of substrate is taken.

In the case of mineral soils, 5 grams of mineral soil is taken and is compacted into a container such as for example a secondary container shown in Figure 1B of the accompanying drawings.

Following compacting of the soil, the soil is passed by way for example of a funnel, into a shaker container. An extractant such as water, and/or sodium bicarbonate preferably in the amount of about 100 mls is then added together with a small portion of an appropriate decolourising powder. The shaker container is then sealed and is agitated by hand for a period of about 2 minutes.

The shaking by hand enables the agitating and mixing and the extraction of nutrients from the soil into the extractant, to take place in a straight forward and efficient manner. If desired, one container can be held in each hand of the user.

Once the shaking or extraction period has expired, the mixture of substrate, extractant and decolourising powder is poured into a primary container.

Where nutrients are being extracted from soiless compost, in one form of the invention 80mls is compacted into a secondary container and this is then passed by way of a funnel into a shaker container. About 120 mls of water is then added together with a portion of decolourising powder. The shaker container is then closed and the container (if desired one in each hand) agitated or shaken for about 2 minutes.

This then results in the nutrients being extracted from the soiless compost or substrate and the shaker container is opened and the matter poured into a primary container.

The addition of the colourising powder is to avoid any colour from the substrate affecting the filtrate to be tested (as will be described hereinafter) especially when some of the testing depends upon comparison between colours and comparison between cloudiness.

Up until this time, numerous means of agitation and shaking have been used, all involving laboratory techniques and machinery which is expensive and time consuming. In particular, up until this time it has been found that it is necessary to agitate to shake the mixture of substrate and extractant for a period of something like 15 minutes. In the present invention however, it has been found that this is not necessary, and that more than adequate nutrients can be obtained for the purposes of testing, by shaking or agitating the containers by hand, for a period of about 2 minutes.

This then is a substantial advance, in that it provides for a straight forward and efficient manner of extraction and avoids the time and expense taken with mechanical and prolonged agitation and extraction.

By way of example, extraction was carried out by hand agitation and shaking, and compared with extraction after laboratory mixing such as laboratory auto analyser. Three samples were tested for nitrate, phosphorus and potassium. The results are set out here below:

COMPARISON OF TEST KIT RESULTS WITH LAB.AUTOANALYSER

| | NITRATE | | PHOSPHORUS | | POTASSIUM | |
|---|---|---|---|---|---|---|
| | Test Kit | Auto Analyser | Test Kit | Auto Analyser | Test Kit | Auto Analyser |
| SAMPLE 1 | 40ppm | 39.2ppm | 1ppm | 1.3ppm | 15ppm | 29ppm |
| SAMPLE 2 | 100ppm | 105ppm | 4ppm | 5.1ppm | 60ppm | 62ppm |
| SAMPLE 3 | 114ppm | 167ppm | 24ppm | 25ppm | 120ppm | 139ppm |

Once the mixture of substrate and extractant has been placed into the primary container, a filter is located within the container.

The filter is located within the container so that the filter isolates an area of filtrate within the container and separates it from the remaining mixture of substrate and solvent. This is shown by way of example in Figure 1 of the accompanying drawings.

Preferably, as shown in Figure 1B of the accompanying drawings, the filter is capable of being formed or folded into a substantially tapered, conical or pyramid type form, so that it extends into the container and isolates an area of filtrate within the primary container which is while being in the primary container, separated from the remainder of the substrate and extractant. Thus for testing purposes, either test paper or droppers or the like can be inserted into the area of clear separated filtrates, which can be used for testing purposes.

The filter is preferably a paper filter which is substantially circular in plan, but which is provided with pleats or folds of a radial nature extending inwardly to a substantial centre point, such that the filter is able to be formed into a substantially conical form for location within the container.

The paper or filtering nature of the filter is such that the equilibrium of the filtrate within the area defined by the filter, within the primary container will be maintained for use in testing.

It has been found that this has been a substantial advantage, in that up until this time and especially in laboratory situations, it has been necessary for funnels and separate filters to be provided, whereby after mixing or extraction, the matter has passed through funnels and filters and has been fed into a container from where the filtrate on its own is contained and used for testing purposes.

By inserting a filter such as that described, into a mixture of substrate and extractant (nutrients having been extracted from the substrate), time and expense are saved in that once the mixture is poured from the shaker container into the primary container, all that is required is for the filter to be folded and neatly inserted into the container whereupon it immediately forms and defines a separated area of filtrate for use in testing.

Thus, this is an advance in so far as time and expense are concerned and it also provides for straight forward operating and effectiveness, especially when incorporated into an arrangement such as the present invention for regular and efficient testing for nutrient levels.

The containers and funnels where used can be of a glass or plastic material as may be desired.

If desired, more than one sample can be tested at a time. The invention will however be described by way of example only, with reference to one sample being tested at a time or one primary container with sample filtrate therein being tested at a time.

The present invention provides for the arrangement to be in a test kit formation, contained in a suitable packing or housing, which can be carried from place to place. The packing or housing is in the form of a case and is provided with the necessary containers, chemicals, testing chemicals, droppers, syringes, containers and the like. Indeed, it can be said that all matter required for the testing of substrate for nutrients, in accordance with the invention, are contained within an overall container for carrying from place to place.

Thus, the invention firstly provides means for the testing of substrate to determine nitrogen or nitrate levels.

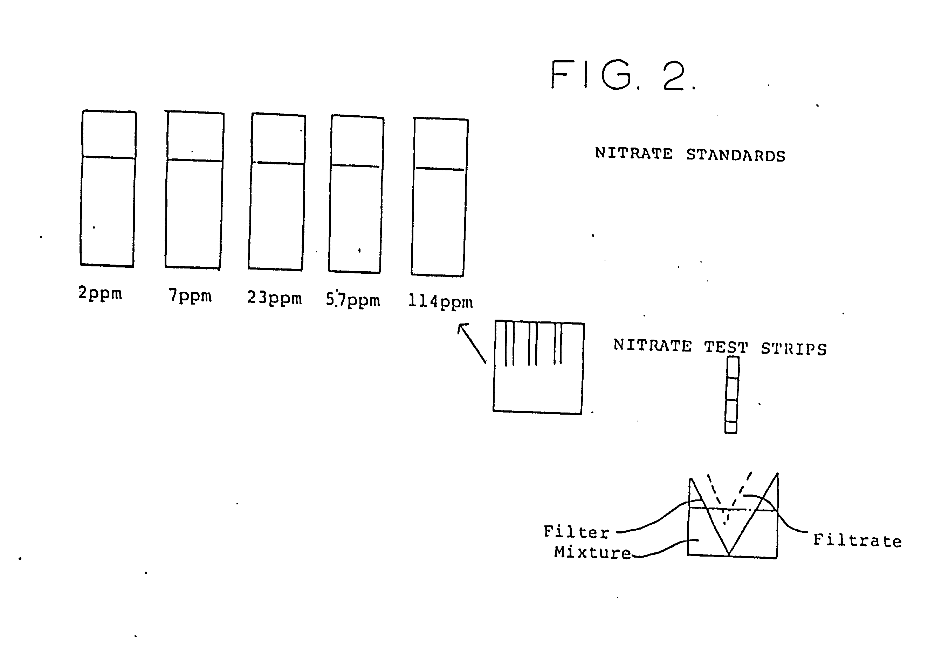

Refererence is made by way of example to Figure 2 of the accompanying drawings.

Firstly, the invention provides for a plurality of nitrogen standards, which are bottles or containers, having varying degrees of nitrogen or matter containing various degrees of nitrogen therein.

These are used for the purpose of comparison with the results from samples.

In one form of the invention the nitrate nitrogen standards are calibrated as:

$NO_3$-N: 2, 7, 23, 57, 114 (parts per million).

Thus, preferably there are five nitrogen standards but if desired, this number can vary. It is however most desirable to have a plurality of nitrogen standards to give variation and a range for comparison purposes.

Thus, the five nitrogen or nitrate standards (hereinafter referred to as "nitrate standards") are open or uncapped and are preferably placed in a stand for the containers.

Nitrate test strips are then taken and one is dipped into the filtrate defined by the filter within the primary container.

The strip is preferably dipped into the solution or filtrate within the filter to wet the squares. It is then shaken to remove excess liquid.

Test strips are then inserted into the nitrate standard containers and after the predetermined or required period of time the sample strips can be compared to determine nitrate levels in the sample being the filtrate.

If desired additional colouring charts or coloured measures can be provided for assistance.

Suitable directions can be provided in association with the invention so that it is clear exactly what desirable nitrate standards are.

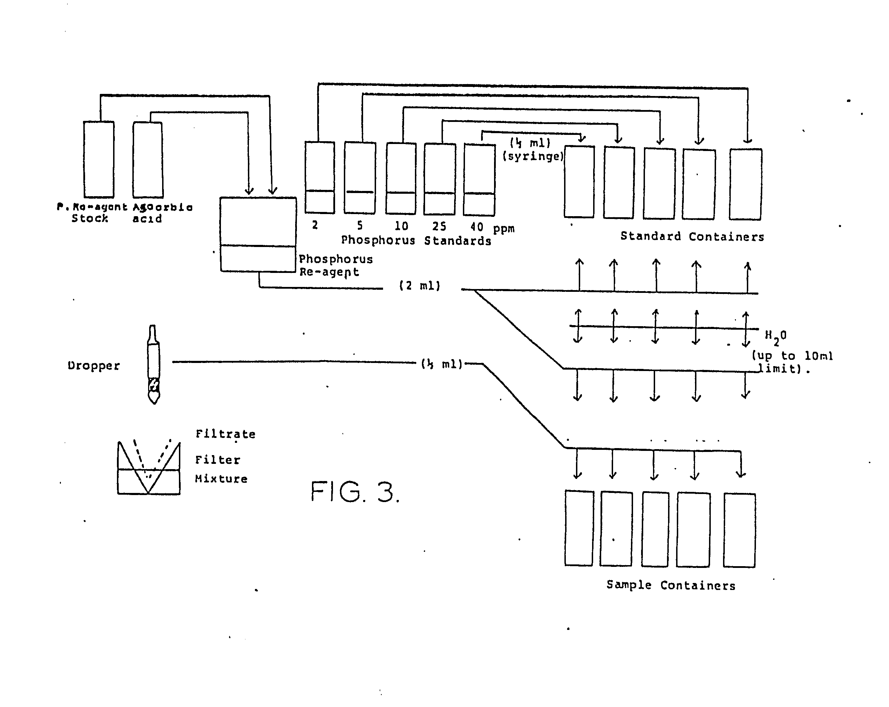

We refer now to Figure 3 of these drawings, which relates to the testing for phosphorus nutrients.

The basic sample material used for testing is the filtrate from the primary container and the filtrate is removed by a dropper from within the area bounded or defined by the filter within the primary container.

A plurality (preferably five) sample containers are provided and these are preferably elongate tubes or vials. These are preferably set up in a stand and the dropper is used to apply ½ ml of filtrate into each sample container.

Water can then be added to each sample container so that there is up to 10 mls in each sample container.

The invention also provides for a phosphorus re-agent which is formed by the combination of phosphorus re-agent stock and phosphorus re-agent tablets and ascorbic acid.

The phosphorus re-agent stock is made up of a mixture of ammonium molybdate, antimony potassium tartartate and sulphuric acid. To form the phosphorus re-agent, which is capable of reacting with phosphorus standards (hereinafter to be described) and samples within the containers, to bring about a clear indication as to phosphorus levels, it is necessary to provide the phosphorus re-agent stock and ascorbic acid separately in that when combined, they do not store.

Thus, a container containing the phosphorus re-agent stock is mixed with the ascorbic acid.

This mixture takes place in a secondary container.

The ascorbic acid is preferably in a tablet form or in the form of a portion of a tablet and it is particularly advantageous to use a concentrated vitamin "C" tablet including ascorbic acid, as this results in quick and speedy dissolving. For example, it is found that it is particularly advantageous to use vitamin "C" REDOXIN (registered trade mark) tablets.

Thus, preferably 25 ml of the phosphorus re-agent stock is mixed in a plastic beaker or secondary container with the ascorbic acid or tablet being a phosphorus re-agent tablet.

This mixture could keep for twelve tests, but will not keep longer than one day.

Phosphorus standards are provided, being containers including phosphorus or matter containing predetermined amounts of phosphrosu which all have phosphorus of predetermined levels.

The levels of phosphorus in phosphorus standards are:

P: 2, 5, 10, 25, 40 (parts per million).

Standard containers are then taken and set up in an appropriate stand, there preferably being five such standard containers. A syringe or dropper is then taken and ½ ml of each phosphorus standard is inserted into a separate standard container.

A further dropper or syringe (or the original one is washed or cleaned) is then used and 2 ml of phosphorus re-agent (as hereinbefore described) is inserted into each of the standard containers and each of the sample containers. Water is then added to each of the standard containers and each of the sample containers to bring the contents of each up to about 10ml.

The containers are then left for a predetermined period of time which is preferably about 10 minutes and after the expiration of that period of time a comparison is made between the colour of each test sample and the colour of the standard containers. These should give an indication as to the nutrient content in the sample containers.

It has been found that it is often difficult to determine the exact colour or degree of difference, and thus if the colour is faint, it has been found to be advantageous to look down the tubes from above, if necessary holding the tubes or containers over a white or light surface to give increased accuracy.

If in use, it is found that the colour of a sample is darker than the highest standard (and is the standard container including a sample from the phosphorus standard having 40 ppm), the test should be repeated, this time using only ½ml of the sample and using 1 ml syringe. The result should however be multiplied by two.

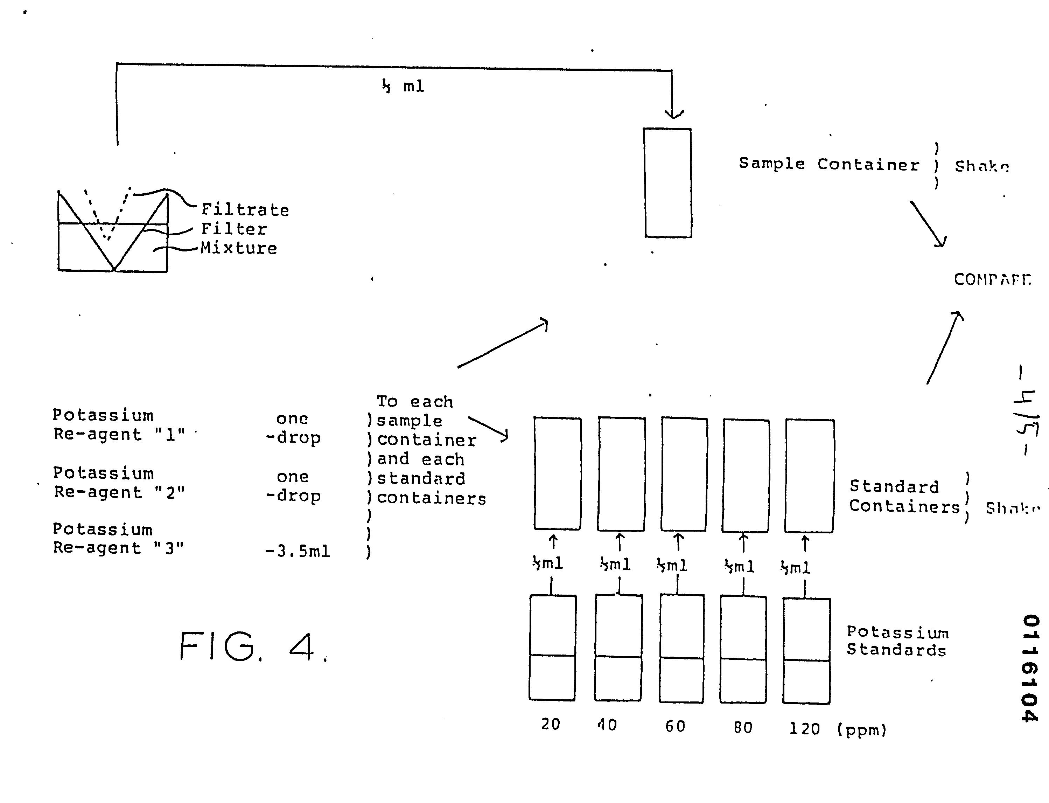

We refer now to Figure 4 of the drawings which relates to testing for the presence of potassium nutrients.

The samples are taken from the filtrate in the primary container the filtrate being bounded by the filter as hereinbefore described and as shown by way of example in the accompanying drawings. A single sample container can be taken and if desired placed in a suitable stand or support.

Preferably by way of a dropper, ½ ml of the filtrate is taken and placed into the sample container. A plurality of potassium standards are provided which are also preferably mounted in a stand or mounting, there preferably being five potassium standards in appropriate bottles or containers.

By way of example the potassium standards are calibrated as follows:

K: 20, 40, 60, 80, 120 (parts per million).

In addition, a plurality of standard containers (preferably five in number) are provided and are mounted in a suitable stand or mounting.

The invention provides for the provision of containers including three potassium re-agents, which we refer to as first potassium re-agent, second potassium re-agent and third potassium re-agent.

The first potassium re-agent is preferably sodium hydroxide. The second potassium re-agent is preferably formaldehyde solution and the third potassium re-agent is preferably sodium tetraphenyl-boron.

These are provided in suitable re-agent containers.

A 1 ml dropper is preferably taken and a ½ ml of the filtrate is placed into the sample container which is preferably a small vial or container.

Using the same 1 ml dropper, ½ ml of each potassium standard is then taken and placed into respective standard containers. Following this, and using again the 1 ml dropper, one drop of first potassium re-agent is added to each container, being each of the sample containers and each of the standard containers. Following this, and again using the 1 ml dropper, one drop of the second potassium re-agent is placed into the sample container and each of the standard containers. The sample container and each of the standard containers are then shaken to mix.

Following this, a syringe or dropper of an appropriate form is taken and 3.5 ml of the third potassium re-agent is added to each of the sample container and standard containers. If desired, pouring out into a beaker may be easier. Each container is then closed or stopped and is mixed up being shaken by hand.

A period of time is then left, and after two minutes, but certainly no longer than ten minutes the cloudiness of the sample, being the matter within the sample container, is compared with the contents of the standard containers. The cloudiness of the matter should be compared.

If difficulty is encountered, it has been found that by shining a light up through the mixture, from the bottom of each container, a clearer indication as to the nature of the cloudiness can be obtained and thus a more accurate comparison formed. It has been found that by using a red light, there are substantial advantages and the degree of cloudiness is further able to be compared.

If the sample is cloudier than the highest standard, being 120 parts per million, the test should be repeated again, taking ¼ ml of the sample and using 1 ml syringe and adding ¼ ml of water. The result should be multiplied by two.

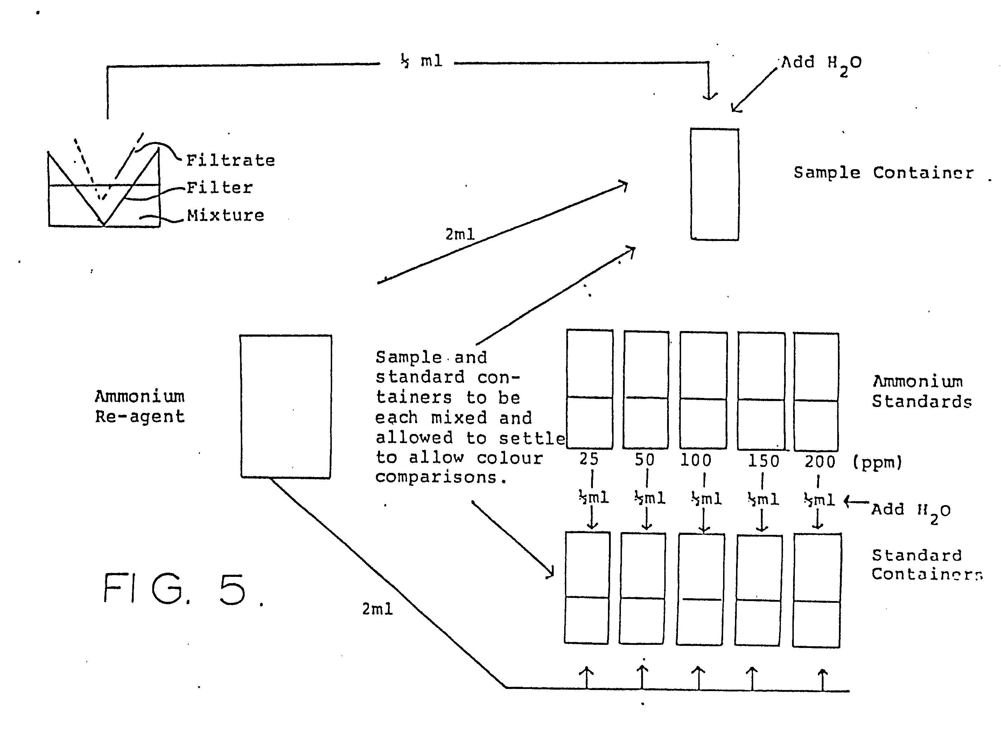

We refer now to Figure 5 of the accompanying drawings, which relates to testing for the presence of ammonium nutrients. As in other tests, the primary container is used and houses the filter and mixture of substrate and solvent, the filter defining an area of clean and clear filtrate within the container. At least one sample container is taken and preferably placed in a suitable holder or rack.

A dropper or applicator is taken and ½ ml of the filtrate is placed into the sample container. Preferably, the dropper used is a 1 ml dropper. The nitrogen standards (as hereinbefore referred to) are used and a plurality, being preferably five, standard containers are inserted into suitable stands so that they can be used. Using a dropper, ½ ml of each nitrogen standard is inserted into respective standard containers.

The sample container and standard containers are then topped up with a solvent such as water, for example until each container has approximately 10 mls of matter within it.

Referring to the ammonium standards, these are in this form of the invention calibrated so that the amounts of ammonium therewithin, are as follows:

$NH_4$-N: 25, 50, 100, 150, 200 (parts per million).

Ammonium re-agent is provided and is in the form of a mixture of red mercuric iodide, potassium iodide and sodium hydroxide. Preferably, the red mercuric iodide and potassium iodide are dissolved in water, the sodium hydroxide also being dissolved in water. The two solutions are then cooled and mixed.

The ammonium re-agent hereinbefore referred to is inserted into the sample container and into each of the standard containers. Preferably, an amount of 2 ml is inserted by way of an appropriate dropper, into the sample container and each standard container. The sample container and standard containers are then inverted a few times or shaken, so as to be mixed.

The sample container and standard containers are then left for a predetermined period of time (for example 5 minutes) and after five minutes the colours of the standard containers can be compared with the colour in the sample container to indicate ammonium nutrient levels in the sample. It will be appreciated that the above are tests to be carried out by equipment provided in a container or kit, and that the invention provides for such tests to be carried out insitu or on site, and avoids as far as possible the lengthy and expensive laboratory testing procedures required up until this time.

Preferred embodiments of the invention are set out hereinbelow as follows:

1. A method of extracting one or more nutrients from substrate, said method including mixing together said substrate with extractant and thereafter shaking or agitating said mixture by hand for a predetermined period of time.

2. A method of extracting one or more nutrients from a substrate including mixing together said substrate with an extractant thereafter shaking or agitating said mixture by hand for a period of up to two minutes.

3. A method as described in paragraph 2, wherein the substrate is compost.

4. A method as described in paragraph 1 or paragraph 3 wherein the extractant is or includes water.

5. A method as described in any one of the preceding paragraphs, wherein the mixture includes 80 mls of compost and 120 mls of water.

6. A method as described in paragraph 1 or paragraph 2, wherein said substrate is mineral soil.

7. A method as described in any one of the preceding paragraphs 1, 2 or 6 wherein the extractant is or includes sodium bicarbonate.

8. A method as described in any one of paragraphs 1,2, 6 and 7, wherein the mixture includes 5 grams of mineral soil and 100 mls of sodium bicarbonate.

9. A method as described in any one of the preceding paragraphs wherein two containers of mixture are agitated or shaken substantially simultaneously.

10. A method as described in any one of the preceding paragraphs, wherein following said shaking or agitation, at least one filter is placed into a container holding said shaken or agitated mixture, the filter(s) being so formed and located that a filtrate of said mixture is provided, and separated from said mixture, within said container.

11. A method of testing a filtrate of a mixture of substrate and extractant for nitrate nutrients, said filtrate having been extracted by a method according to any one of the preceding paragraphs 1 to 10 including:

1. inserting at least one strip of nitrate test paper into said filtrate and thereafter withdrawing same;
2. inserting at least one strip of nitrate test paper into at least one nitrate standard (as herein defined) and thereafter withdrawing same;
3. waiting for a predetermined period of time
4. thereafter comparing the colours of said test strips.

12. A method as described in paragraph 11, wherein a plurality of nitrate standards (as herein defined) are provided, said method including inserting at least one test strip into each of said nitrate standards and at least one test strip to said filtrate; thereafter withdrawing same and waiting a predetermined period of time; thereafter comparing colours of said strip from said filtrate with the colour of said strip(s)from said nitrate standard.

13. A method of testing a filtrate of a mixture of substrate and extractant for presence of phosphorus nutrients, said filtrate having been extracted according to the method as described in any one of the preceding paragraphs 1 to 10, including:

1. taking one or more standard containers;
2. taking one or more test containers;
3. inserting 1 ml of said filtrate into said one or more sample conatiners;
4. inserting ½ ml of phosphorus standard (as herein defined) into said one or more standard containers;
5. inserting 2 mls of phosphorus re-agent into each container.
6. waiting a predetermined period of time;
7. thereafter comparing the colour in said one or more test containers with the colour in said one or more sample containers.

14. A method as described in paragraph 13, wherein a comparison in colour is obtained by looking down through said containers.

15. A method as described in paragraph 14, wherein said containers are viewed over a white surface.

16. A method as described in any one of the preceding paragraphs 12 to 15, wherein the containers are tubes.

17. A method as described in any one of the preceding paragraphs 12 to 15, wherein five test containers and five sample containers are provided.

18. A method as described in any one of the preceding paragraphs 13 to 17, wherein the ½ ml of phosphorus standard (as herein defined) is inserted into each container, water is thereafter added so as to bring the level in each container up to 10 mls.

19. A method as described in any one of the preceding paragraphs 13 to 18, wherein the phorphorus re-agent includes absorbic acid.

20. A method as described in paragraph 19, wherein the absorbic acid is in the form of at least part a vitamin C tablet.

21. A method of testing for the presence of potassium nutrients in a filtrate extracted from a mixture of substrate and extractant, according to the method described in any one of the preceding paragraphs 1 to 10, including:

1. inserting ½ ml of filtrate into one or more sample containers.
2. inserting ½ ml of potassium standard (as herein defined) into one or more standard containers.
3. inserting a predetermined amount of first potassium re-agent into each conatiner.
4. inserting a predetermined amount of second potassium re-agent into each container.
5. shaking each container so as to mix the contents thereof.

6. inserting 3.5 mls of third potassium re-agent into each container and thereafter mixing contents of each container.

7. allowing said containers to sit for a predetermined period of time.

8. after a predetermined period of time comparing cloudiness of contents of said one or more test containers with cloudiness of contents of said one or more standard containers to determine standard of potassium nutrients present in said filtrate.

22. A method as described in paragraph 21, wherein a light is applied through each container to assist in determining degree of cloudiness.

23. A method as described in paragraph 22, wherein the light is a red light.

24. A method as described in any one of the preceding paragraphs 21 to 23, wherein said first potassium re-agent includes sodium hydroxide.

25. A method as described in any one ofthe preceding paragraphs 21 to 24, and wherein said second potassium re-agent includes formaldehyde.

26. A method as described in any one of the preceding paragraphs 21 to 25, wherein said third potassium re-agent includes sodium tetraphenyl boron.

27. A method as described in any one of the preceding paragraphs 21 to 26 wherein after addition of the third potassium re-agent, contents of said containers are compared after expiration of 2 minutes.

28. A method as described in paragraph 27, wherein comparison is made no longer than 10 minutes after said mixing.

29. A method of testing for the presence of ammonium nutrients in a filtrate of a mixture of substrate and extractant, said filtrate being extracted from said mixture of substrate and extractant by a method according to any one of the preceding paragraphs 1 to 10, including:

1. inserting ½ ml of said filtrate into a sample container.
2. inserting ½ ml of nitrogen standard (as herein defined) into one or more standard containers.
3. inserting a predetermined amount of water into each container.
4. inserting 2 ml of ammonium re-agent into each container and thereafter closing and shaking same.
5. after a predetermined period of time comparing colour in said one or more test containers with colour in one or more standard containers to determine ammonium nutrient presence.

30. A method as described in paragraph 29, wherein following the addition of ammonium re-agent to each container, and the mixing thereof, said comparison takes place after a period of five minutes.

31. A method as describedin either of the preceding paragraphs 29 and 30, wherein a plurality of containers of nitrogen standard (as herein defined) are provided.

32. A method as described in paragraph 31, wherein ½ ml is taken from each nitrogen standard (as herein defined) and placed into a respective standard container.

33. A method as described in any one of the preceding paragraphs and wherein the growing medium is compost.

34. A method as described in paragraph 33 and wherein the compost is soiless compost.

35. A method as described in any one of the preceding paragraphs wherein the extractant is water.

36. A method as described in any one of the preceding paragraphs wherein said filter is substantially conical or tapered in use and in location within said container.

37. A method as described in paragraph 36, wherein the filter is a paper filter.

38. An arrangement for testing growing medium for one or more nutrients including:

1. at least open ended primary container ;
2. a mixture of growing medium and extractant within said primary container;
3. at least one substantially conical or tapered filter being located within said container;
4. a filtrate being defined and isolated by said filter within said container;

5. said filtrate being from said mixture of growing medium and extractant.

6. said filtrate being separated from said mixture of growing medium and extractant but within said container, by location of said filter.

39. Apparatus for use in testing growing medium for one or more nutrients, including a casing or housing provided with or housing and locating:

1. one or more shaker containers;

2. at least one primary container;

3. at least one filter capable of being formed into a substantially tapered or conical shape or form in use;

4. a plurality of secondary containers or tubes such as to provide at least one sample container and one standard container for each nutrient test;

5. a plurality of nutrient standards (as herein defined);

6. a plurality of nitrate test strips;

7. phosphorus re-agent stock (as herein defined);

8. ascorbic acid;

9. first, second and third potassium re-agents (as herein defined);

10. one or more applicators.

40. Apparatus as described in paragraph 49, wherein at least one applicator is calibrated and/or provided or formed with a disposable or replaceable tip portion.

41. An apparatus as described in paragraph 39 or paragraph 40 including racks for holding said one or more standard containers and said one or more sample containers.

42. An apparatus as described in any one of the preceding paragraphs 39, 40 or 41, wherein said containers are tubes.

It should be appreciated that modifications may be made to the invention without departing from the scope or spirit thereof, as defined by the appended claims.

WE CLAIM:

1. A method of extracting one or more nutrients from substrate, said method including mixing together said substrate with extractant and thereafter shaking or agitating said mixture by hand for a predetermined period of time.

2. A method of extracting one or more nutrients from a substrate including mixing together said substrate with an extractant and thereafter shaking or agitating said mixture by hand for a period of up to two minutes.

3. A method as claimed in claim 1 or claim 2, wherein following shaking or agitation, at least one filter is placed into a container holding said shaken or agitated mixture; said at least one filter being so formed and located that a filtrate of said mixture is formed and separated from said mixture, within said container.

4. A method of testing a filtrate of a mixture of substrate and extractant for nitrate nutrients, said filtrate having been extracted by a method according to any one of the preceding claims 1 to 3, including:

1. inserting at least one strip of nitrate test paper into said filtrate and thereafter withdrawing same.

2. inserting at least one strip of nitrate test paper into at least one nitrate standard (as herein defined) and thereafter withdrawing same.

3. waiting for a predetermined period of time.

4. thereafter comparing the colours of said test strips.

5. A method as claimed in claim 4, wherein a plurality of nitrate standards (as herein defined) are provided, said method including inserting at least one test strip into each of said nitrate standards and at least one test strip into said filtrate; thereafter withdrawing same and waiting a predetermined period of time; thereafter comparing colours of said strip from said filtrate with the colour of said at least one strip from said nitrate standard.

6. A method of testing a filtrate of a mixture of substrate and extractant for presence of phosphorus nutrients, said filtrate having been extracted from a mixture of substrate and extractant according to a method as claimed in claim 1 or claim 2, said method including:

1. taking one or more standard containers;

2. taking one or more test containers;

3. inserting 1 ml of said filtrate into said one or more sample containers;

4. inserting ½ ml of phosphorus standard (as herein defined) into said one or more standard containers;

5. inserting 2 mls of phosphorus re-agent into each container;

6. waiting a predetermiend period of time;

7. thereafter comparing the colour in said one or more test containers with the colour in said one or more sample containers.

7. A method of testing for the presence of potassium nutrients in a filtrate extracted from a mixture of substrate and extractant, said filtrate having been extracted from said mixture of substrate and extractant by a method as claimed in claim 1 or claim 2, said method including:

1. inserting ½ ml of filtrate into one or more sample containers;

2. inserting ½ ml of potassium standard (as herein defined) into one or more standard containers;

3. inserting a predetermined amount of first potassium re-agent into each container;

4. inserting a predetermined amount of second potassium re-agent into each container;

5. shaking each container so as to mix the contents thereof;

6. inserting 3.5 mls of third potassium re-agent into each container and thereafter mixing contents of each container;

7. allowing said containers to sit for a predetermined period of time;

8. after a predetermined period of time comparing cloudiness of contents of said one or more test containers with cloudiness of contents of said one or more standard containers to determine standard of potassium nutrients present in said filtrate.

8. A method of testing for the presence of ammonium nutrients in a filtrate of a mixture of substrate and extractant, said filtrate having been extracted from said mixture of substrate and extractant by a method according to claim 1 or claim 2, said method including:

1. inserting ½ ml of said filtrate into a sample container;

2. inserting ½ ml of nigrogen standard (as herein defined) into one or more standard containers;

3. inserting a predetermined amount of water into each container;

4. inserting 2 ml of ammonium re-agent into each container and thereafter closing and shaking same;

5. after a predetermined period of time comparing colour in said one or more test containers with colour in one or more standard containers to determine ammonium nutrient presence.

9. An arrangement for testing growing medium for the presence of one or more nutrients, according to the method claimed in any one of the preceding claims 4 to 8, said arrangement including:

1. at least open ended primary container;

2. a mixture of growing medium and extractant within said primary container;

3. at least one substantially conical or tapered filter being located within said container;

4. a filtrate being defined and isolated by said filter within said container;

5. said filtrate being from said mixture of growing medium and extractant;

6. said filtrate being separated from said mixture of growing medium and extractant but within said container, by location of said filter.

10. Apparatus for use in testing growing medium for the presence of one or more nutrients, according to the method claimed in any one of the claims 4 to 8, including a casing or housing provided with or housing and locating:

1. one or more shaker containers;

2. at least one primary container;

3. at least one filter capable of being formed into a substantially tapered or conical shape or form in use;

4. a plurality of secondary containers or tubes such as to provide at least one sample conatiner and one standard container for each nutrient test;

5. A plurality of nutrient standards (as herein defined);

6. a plurality of nitrate test strips;

7. phosphorus re-agent stock (as herein defined);

8. ascorbic acid;

9. first, second and third potassium re-agents (as herein defined);

10. one or more applicators.

substrate
compact
(80mls)
(Measure of decolourising powder)
$H_2O$
(120mls)
Shaker
Shake
2 mins.
Filtrate
Filter
Mixture
Soil
(5 gms)
100mls
Sodium Bicarbonate

FIG. 1.

FIG. 1A

FIG. 1.B.

FIG. 2.
NITRATE STANDARDS
NITRATE TEST STRIPS
Filtrate
Filter
Mixture
114ppm
57ppm
23ppm
7ppm
2ppm

P. Re-agent Stock
Ascorbic acid
Phosphorus Re-agent
2
5
10
25
40 ppm
Phosphorus Standards
(½ ml) (syringe)
Standard Containers
(2 ml)
$H_2O$ (up to 10ml limit).
(½ ml)
Sample Containers
Dropper
Filtrate
Filter
Mixture

FIG. 3.

½ ml
Sample Container
Shake
Filtrate
Filter
Mixture
COMPARE
Potassium Re-agent "1"
one -drop
Potassium Re-agent "2"
one -drop
Potassium Re-agent "3"
-3.5ml
To each sample container and each standard containers
Standard Containers
Shake
½ml
Potassium Standards
20
40
60
80
120
(ppm)

FIG. 4.

½ ml
Add $H_2O$
Filtrate
Filter
Mixture
Sample Container
2ml
Ammonium Re-agent
Sample and standard containers to be each mixed and allowed to settle to allow colour comparisons.
Ammonium Standards
25
50
100
150
200
(ppm)
½ml
½ml
½ml
½ml
½ml
Add $H_2O$
Standard Containers
2ml


FIG. 5.

European Patent Office

# EUROPEAN SEARCH REPORT

0116104

Application number

EP 83 10 1338

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | DE-C- 834 601 (A. KAWE) * Whole document * | 1 | G 01 N 33/24<br>G 01 N 31/00 |
| A | | 3,6,7 | |
| | --- | | |
| A | DE-A-1 951 112 (F. ZIMMERMANN) * Claim 1; page 1, line 4 - page 2, line 21 * | 1,3 | |
| | --- | | |
| A | G. JANDER et al. "Lehrbuch der analytischen und präparativen anorganischen Chemie", 8th edition, chapter 4: "Analytischer Teil", 1969, S. HIRZEL VERLAG, Stuttgart, pages 151-160, 183-188, 280, 364 * Page 153, no. 5: "Nachweis mit Lunges Reagenz"; page 158, no. 7: "Nachweis mit Ammoniummolybdat"; page 185, no. 7: "Nachweis mit 'Kalignost'"; pages 187-188, no. 8: "Nachweis mit 'Nesslers Reagenz'"; page 280, no. 1: "H2O, Weinsäure"; page 364, paragraph 5 * | 4,6-8 | TECHNICAL FIELDS SEARCHED (Int. Cl. 3)<br>G 01 N 31/00<br>G 01 N 33/24 |
| | --- | | |
| A | DE-B-2 806 047 (W. TEPE) * Claim; column 1, lines 31-36 * | 4,6-8 | |
| | ----- | | |

The present search report has been drawn up for all claims

| Place of search | Date of completion of the search | Examiner |
|---|---|---|
| BERLIN | 26-09-1983 | HOFMANN D G |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82